Europäisches Patentamt

European Patent Office

Office européen des brevets

(11) Publication number: **0 196 396 B1**

(12) **EUROPEAN PATENT SPECIFICATION**

(45) Date of publication of patent specification: **16.01.91**

(21) Application number: **85830081.7**

(22) Date of filing: **01.04.85**

(51) Int. Cl.⁵: **A 61 B 5/00, A 61 B 5/08, G 01 F 1/10**

(54) Portable breathing monitor for telemetric measurement by a central processing station.

(43) Date of publication of application:
**08.10.86 Bulletin 86/41**

(45) Publication of the grant of the patent:
**16.01.91 Bulletin 91/03**

(84) Designated Contracting States:
**AT BE CH DE FR GB IT LI NL SE**

(56) References cited:
**CH-A- 460 244**
**DE-A-1 466 834**
**DE-A-1 804 160**
**DE-A-2 313 131**
**DE-B-1 566 066**
**FR-A-2 145 020**
**US-A-3 523 529**
**US-A-3 741 208**

(73) Proprietor: **COSMED S.r.l.**
**Via del Tempio degli Arvali, 41/L**
**I-00148 Roma (IT)**

(72) Inventor: **Brugnoli, Siro**
**Via Valdieri 31**
**I-00135 Roma (IT)**

(74) Representative: **Mascioli, Alessandro, Prof.Dr.**
**c/o A.N.D.I. Associazione Nazionale degli Inventori Via Urbana, 20**
**I-00184 Roma (IT)**

Courier Press, Leamington Spa, England.

EP 0 196 396 B1

## Description

The present invention concerns a device for the survey of the breathing ventilation and of the oxygen consumption for sending radio signals to a fixed reception and elaboration unity comprising a volume meter, an oxygen concentration meter, a control and a radio transmitter module of small dimensions and weight so as to be portable for to the person being examined.

The meters known in the art do not allow a survey in the "real" conditions, i.e. during work or sport, and therefore static laboratory analysis must be performed.

In these situations it is impossible to obtain such conditions that are however necessary for a valuation of the breathing functionality in "limit" conditions like the ones of an athlete in a contest or during training, or of a person working in such ambiental conditions that could prove to be dangerous for his health (smoke, smog, noise, heat, etc.).

It is known that breathing ventilation (VE) means the air volume expired by a person in one minute; while oxygen consumption VO2 means the volume of oxygen consumed in one minute while breathing.

The VO2 is given by the product of the ventilation minute multiplied by the difference between the inhaling and expiration oxygen concentration.

The average expiratory concentration must be calculated because during the expiration the oxygen percentage is not constant.

The VO2 calculation is completed with the survey of the ambiental pressure, temperature and moisture values that allow to bring back the measure of the VO2 to the "standard" conditions provided for the gaseous volumes (20°C, 760 mmHg, 0% relative moisture).

The volume meters actually known are of the kind:

1) closed circuit
2) open circuit.

Between the closed circuit volume meters the most used are:

a) container or piston.

In this case, a cylinder is moving inside another one in function of the gas that is let in and out. The sealing between the two cylinders is obtained by membranes or even immersing the movable cylinder in a liquid contained in the second cylinder.

b) Bellows.

The volume is obtained by the capacity variation of the bellow. The most used are the angular variation bellows like the ones used to blow on the fire.

The volume meters with open circuit actually most used are:

a) pneumotacograph of Fleisch and Lilly.

Both are based on the principle of the tube of Venturi.

b) Hot wire.

Consisting in an element whose electric features (e.g. the resistance) vary according to the speed of the air flow that strikes it.

c) Turbine or fly.

Performs a number or revolutions proportional to the volume that strikes it.

The turbine device already known shows the disadvantage due to the inertia moment due to the rotation of the propeller around the axis.

Infact, the turbine continues to revolute, even when the flow striking the same is annulled, for a time period called "of arrest", being inversely proportional to the resistance and to the frictions of the system.

It is not possible to increase said frictions beyond determined limits for reducing the time of arrest, because to each friction increase corresponds a minimum flow (threshold) increase, that places the turbine into rotation, and below which no good signal is obtained because the turbine remains still.

It is evident that the "ideal" turbine meter is the one having a threshold flow and a time of arrest being nul.

The most used devices for measuring the oxygen concentration may be grouped in three main classes:

1) thermoparamagnetic (or at magnetic wind);
2) magnetodynamic (or pure magnetic);
3) electrochemical, in turn divided into polarographic and galvanic.

A further classification of the analyzers may consist in using as a reference parameter the response time (tr) of the instrument:

1) QUICK analyzers (tr<200 ms);
2) SLOW analyzers.

The difference between the two classes is not limited to the response time, as said time has a considerable influence on the other technical features.

The stability and the exactness of a SLOW analyzer, e.g. are always considerably higher than those of a QUICK analyzer.

Usually, when a high stability is requested, it is necessary to keep the instrument always working, around the clock, if it is a QUICK one, while only a few minutes are enough for reaching the speed conditions if it is a SLOW analyzer.

This is due to the different chemical and physical features of oxygen, that are utilized in the two kinds of instruments to obtain the value of the concentration.

Furthermore, the QUICK analyzers are extremely sensitive to the pressure, temperature and moisture variations that take place inside the measuring chamber.

E.g., all known analyzers want the gas flow to be measured to be constant (0.5 or 1 l/min).

Otherwise there will be enormous measuring errors.

It can therefore be easily understood that also the auxiliary devices, like the aspiration pump, must have the features of exactness and stability.

All this means a clear difference between the two kinds of analyzers, also for what concerns the cost and the practicity in use.

Usually, a QUICK analyzer is five to ten times more expensive that the SLOW one. Furthermore, due to the particular functioning, mainly for what concerns the stability, the QUICK analyzer is not suited for being carried about while functioning.

Also the fact that the QUICK analyzer is heavier and takes away more space than the SLOW one, should be considered.

The only advantage of a QUICK analyzer is, therefore, the fact that measures can be taken in a real time.

In the medical use this means that it will be possible to "follow" such phenomena that quickly vary during a determined period, without "loosing" information.

For what concerns the methods actually used for the survey of the oxygen percentage, the same concern:

a) the momentary measuring of the oxygen percentage present in the expired gas and the following calculus of the average value;

b) the direct measuring of the oxygen average expiratory percentage effected by means of a "mechanical mediator".

For momentary measuring QUICK analyzers are used, the response time of which does not exceed 150/200 msec.

The gas sampling will be effected near the person's mouth.

On the contrary, the direct measuring of the average oxygen value may be effected with quick as well as with slow analyzers.

For obtaining the average value, the expired volume will be sent into a chamber for the mixing of the expired gases that works as "mechanical mediator".

When the measuring of the oxygen percentage inside said chamber is effected by means of a quick analyzer, the capacity of the same does not represent particular difficulties, because the quickness of the analyzer assures a correct measure independently from the volume expired by the person.

If, on the contrary, a slow analyzer is used, the chamber's capacity will have to be determined according to the response time of the analyzer and of the ventilation.

Usually, the oxygen consumption measuring is effected at regular time intervals (e.g. each 30 seconds). This means that between one measure and the other, a determined gas volume must be collected in the chamber, representative, in terms of an average oxygen percentage, of the time interval being examined.

Usually, "turnover" shows how many times in one minute a volume equal to the one of the chamber has passed inside the same, so as to obtain the so-called "washing speed of the chamber".

So as to avoid that the concentration measuring during one calculation interval be influenced by the oxygen percentage values relating to the precedent interval, it is necessary to effect a minimum turnover number Tmin that assures the "renewal" of the mixing chamber.

On the other hand, the response time of the analyzer is such as to impose a limit also for the maximum turnover number Tmax.

In other words, for a too low ventilation the renewal of the chamber is not quick enough and therefore the oxygen percentage is influenced by the values of the aforegoing interval.

The ventilation being an independent variant, the only term on which it is possible to act is the capacity C of the mixing chamber. The same must be such as to verificate the relation:

Tmin x C < VE <Tmax x C.

Once the values of Tmin and Tmax are fixed at a determined capacity value of the chamber C, it can be seen that the ventilation may vary between a minimum VEmin and a maximum VEmax.

It will be necessary to modify the value of C according to VE for removing this obstacle.

This infact is the main principle on which the so-called "dynamic" chambers at variable capacity are based.

The known devices use static as well as dynamic chambers.

Usually the capacity of the same varies between about two to twelve litres.

A further method for the measuring of the average expiratory concentration is known, and it consists in the continuous taking of a sample (e.g. 1/100) of the expired gas that will be sent into a micro mixing chamber. This allows to considerably reduce the capacity and therefore the dimensions of the chamber.

As during the expiration phase the oxygen percentage is not constant, the sampling flow shall be proportional to the expiration one so that the sample taken from the mouth be representative of the expired volume.

This method may be compared to the one realized by means of a fix mixing chamber to which all the expired volume is sent. It is evident that the same limits are considered for the fix chamber, considering that between the two methods there is only a capacity difference of the chambers due to the sampling.

Usually the systems for measuring the VE and the VO2 are encumbersome and heavy devices, so that the use thereof must be limited to the laboratory.

For what concerns the so-called "portable" systems, there are instruments that weigh about 8 kg, obtained by the miniaturizing of some fundamental elements; but the weight and dimensions don't make them suitable for performing any sport activity therewith, except riding a horse or a bicycle.

Furthermore the method for the calculation of the oxygen percentage is the one of the fix mixing chamber. Therefore, this is a traditional system, and the weight and dimensions thereof do not solve the problem of carrying it about during its use.

Said system is completed by a microcomputer and by a small tape recorder that allows a late data elaboration.

The mentioned difficulty of measuring the variable composition of the expired gases is examined in the SIEMENS patent CH-A-460 244, wherein a gas sampling system is described in which the sampled flow is proportional to the expiration flow. A membrane aspiration pump is described, the capacity thereof being controlled by the signal coming from a device for measuring the flow of the kind of a pneumotachograph. However, such invention does not solve the problem of the turnover control and therefore the volume for each time unit flowing through the gas mixing chamber can not be controlled, but it depends on the ventilation value.

DE-A-2 313 131 of Klaus Junginger describes a device for measuring the flow of the rotating kind (like a turbine or small propellers), as well as some methods for the counting of the revolutions of the mobile equipment. In particular, methods are listed based on apposite signs placed on the propeller, deposits of magnetic or radioactive materials. At the same time devices for the counting of the revolutions are shown. The possibility is also mentioned to make use of said outlet signal of the device for measuring the flow for controlling the capacity of an eventual pump. In this invention, however, the problem of correcting the flow signal is not considered for eliminating the counting due to the inertia of the rotating element.

The need of a telemetric transmission of physiological signal, in particular of those connected to the cardiorespiratory functionality of the patient in free movement, for performing observations in conditions of natural efforts is described in DE-A-1 466 834 of HELLIGE. This invention, however, still needs considerable methodological and technological developments so as to make the system easily transportable from a point of view of weight and encumbrance, said system consisting of a device for measuring the flow, a sampling device and an analyzer of the expired gas.

It is the aim of the present invention to reduce the dimensions and the weight of a device for the survey of the breathing ventilation and of the oxygen consumption so as to be portable for the person to be examined during work or sport activity.

The aim set forth is reached by means of a device characterized in a portable survey and transmission unity for sending radio signals to a fixed reception and elaboration unity.

The portable unit on the person to be examined comprises a breathing mask with a tube wherethrough expired and inhaled air flows, supporting the devices for the survey of the ventilation and temperature signals as for the expiratory volume sampling.

Said signals are then directed to a radio control and transmission module; these also being portable for the person in a belt placed in such a way that it does not disturb his physical activity.

The control module activates the circuit that realizes the special method of sampling of the expiratory volume and of the turnover management, thus also obtaining the signal relative to the oxygen percentage.

The transmitter sends the signals to the fixed reception and elaboratory unit.

The present invention is explained more in detail hereinbelow according to the attached drawings in which a preferred embodiment is shown.

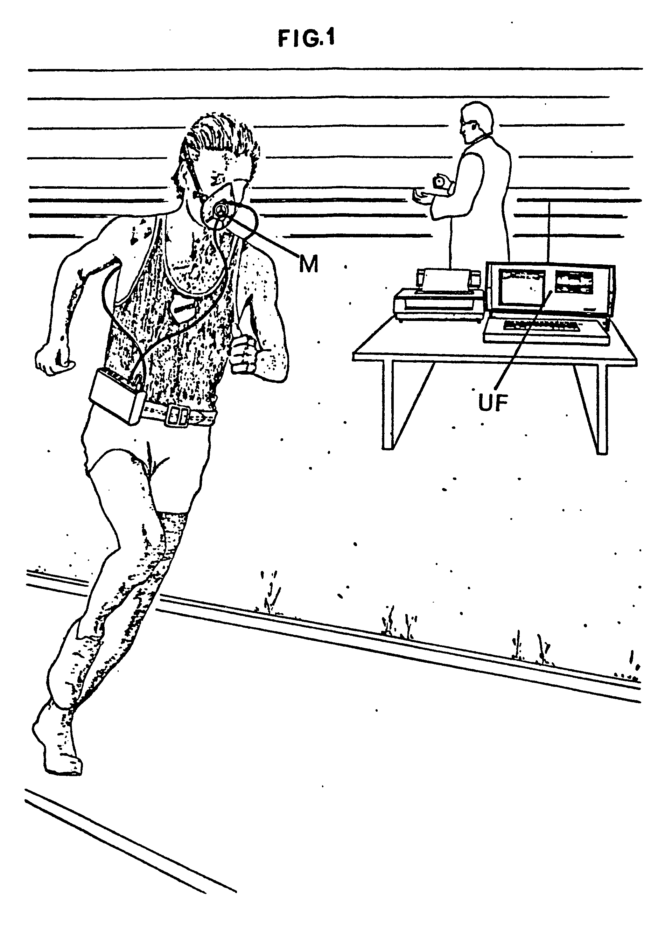

Figure 1 shows a perspective view of a device used by an athlete while running.

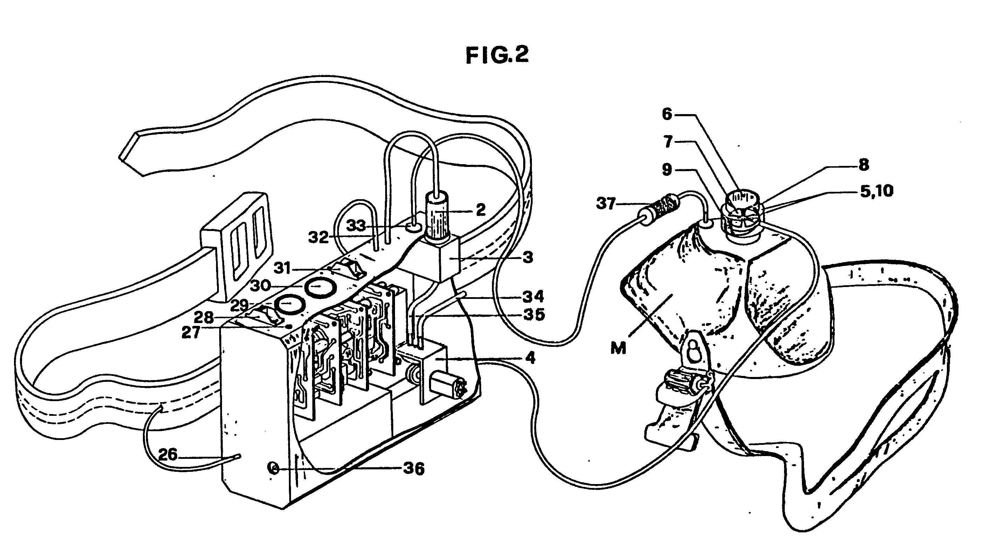

Figure 2 shows a perspective view of a cross section of a device for the survey and transmission of data.

Figure 3 shows in detail one of the possible functioning schemes.

The figures show a portable device for the survey of the breathing ventilation and of the oxygen consumption for sending radio signals to the fix reception and elaboration unity UF, comprising an oxygen meter 2 with polarographic electrode, a sampling pump 4, a photo diode sender 5, a volume survey system 6, a turbine 7, a temperature sensor 8, a pressure transducer 9, a photo surveyor 10, an oxygen annulment 20, an oxygen amplification adjuster 21, a radio transmission antenna 26, an accumulator charge level indicator led 27, a starting switch 28, an automatic gauge switch 31, a mask connection cable 32, an inlet 33 of the mixing chamber, crossed by the sampled volume aspired by the sampling pump, a connector 36 for accumulator charge, a moisture absorber 37.

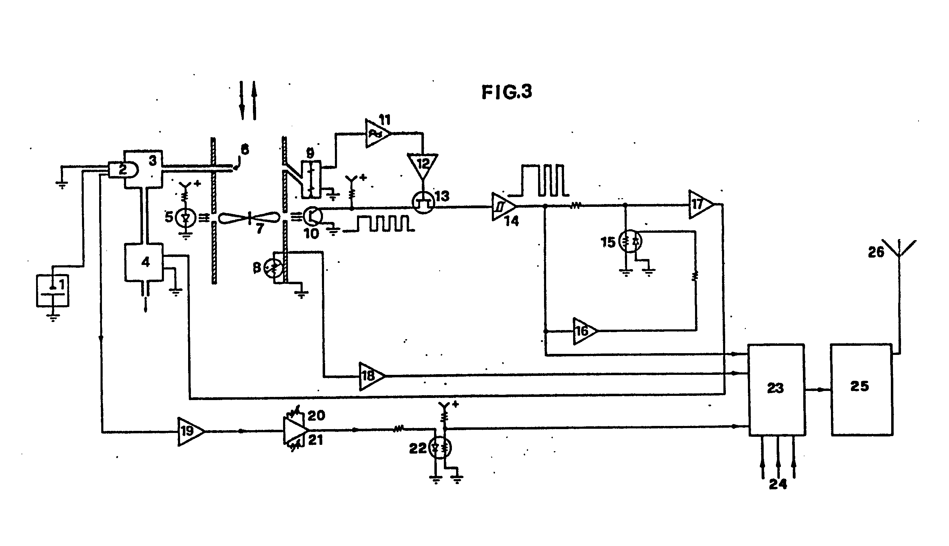

For what concerns the scheme of figure 3, beyond the already mentioned details, there are shown; a circuit 1 of oxygen electrode polarizer, an electronic filter 11, a comparator 12, a transistor FET 13, a Schmitt TRIGGER 14, a photo resistor 15, an integrator 16, a power amplifier 17, a (temperature) amplifier 18, an (oxygen) amplifier 19, a photo resistor 22, a radio frequency modulator 23, auxiliary inlets 24 (EGG, SaO2, ect.), a radio transmitter 25.

For what concerns the turnover management, the present invention acts on the value of $F = Fe/Fc$, where Fe is the expiratory flow and Fc is the sampling flow, instead on $C = $ chamber capacity, thus obtaining a mechanical mediator; the functioning thereof may overlap—for what concerns the result—the one that could be obtained varying the capacity of a dynamic chamber being F times bigger.

The measuring of the ventilation will be effected, according to the present invention, by means of a high precision traditional turbine 7 and pressure transducer 9, that are suited for a practical and economical recognition of the nul flow that eliminates the inconveniences due to the "arrest time" of said turbine.

Inside the tube containing turbine 7, a pressure transducer 9 (e.g. a microphone) will be inserted, for recognizing in a nul time the flow conditions different from zero.

Transducer 9 has an outlet signal being proportional to the pressure and therefore to the air flow striking the same with a response time less than 1 msec.

In other words, said transducer is used as ON-OFF indicator for recognizing the flow annulment and thus correct the signal the turbine contributes sending during the arrest time.

Thus the main error source will be eliminated.

For what concerns the functioning of the device according to the present invention, the person is connected, by means of the mask M with a tube wherethrough expired and inhaled air flows, to a gaseous volume survey system 6, consisting in a turbine 7 rotating at a speed proportional to the air volume striking the same.

The photo diode system 5 and the photo surveyor 10 allows to generate an impulse train proportional to the revolution number of the turbine.

As already shown, at the end of each expiration and inhaling, when the air flow is annulled, the impulses obtained due to the inertia moment of turbine 7 are suppressed by means of a correction circuit consisting in a pressure transducer 9 that immediately reveals (less than 1 msec) the flow annulment.

The outlet signal of transducer 9 filtered by circuit 11 is sent to comparator 12 that controls transistor FET 13, the ON-OFF functioning thereof allowing or preventing the transmission of the impulse train to an amplifier Schmitt trigger circuit 14.

The filter 11 also allows the elimination of the impulses generated during the inhaling stage.

The impulse train present at the Schmitt trigger 15 outlet therefore represents "the correct expiratory volume" that will be sent of modulator 23 to which the radio transmitter 25 is connected.

The same signal will be sent to a power amplifier 17 that controls the capacity of the sampling pump 4 and to an integrator 16.

Said integrator, by means of the photoresistor 15, controls the level of the inlet signal of the power amplifier 17.

The sampling flow of pump 4 is always proportional to the expiratory flow according to an amplification factor controlled by integrator 16, which therefore has the function of "automatic gain control".

The sample volume of pump 4 shall be sent to a micro mixing chamber 3 inside which there is a polarographic electrode 2 for the measuring of the oxygen percentage.

The suitably amplified outlet of electrode 2 is connected to modulator 23 and to transmitter 25.

The system is completed with a sensor 8 for the survey of the expired gas temperature, the outlet thereof being amplified 18 and sent to modules 23 and 25.

In a variant the present invention also provides an inlet 24 and the transmission of auxiliary signal coming from other instruments, like EGG (electrocardiographic signal) or SaO2 (signal for the oxygen blood saturation).

In a possible variant the present invention provides the presence, instead of or in addition to transmitter 25, of a tape recorder or similar or a digital recorder, with electronic memory, that will allow the late data analysis.

The main advantages of the present invention are in the following:

1) the presence, in the device, of a slow oxygen analyzer that guarantees, with respect to a quick one, a greater exactness and stability in measuring, beyond a smaller "criticity", at a cost five to ten times lower;

2) the concept of sampling by means of a pump with variable capacity according to the expiratory flow allows to analyze, beyond the average value of the oxygen percentage, also the one relative to a "portion" of expiration. For analyzing "End breathing" values, i.e. those oxygen percentage values at the end of each expiration and that are very important in the analysis of the breathing functionality, it will be enough to operate the sampling pump during the end of expiration, which can be easily recognized by the signal coming from the volume meter. An analysis of this kind has been possible until now only by means of very expensive quick analyzers.

3) The possibility of using a slow analyzer without renouncing to the advantages offered by the quick ones has allowed a great miniaturizing, that can not be obtained by any quick analyzer in commerce.

4) The controlled turnover sampling makes the fix capacity micro mixing chamber equivalent to a dynamic chamber at variable capacity, with the advantage of a considerable dimension reduction.

5) The controlled turnover annuls the limitation already listed for the slow analyzers, i.e. the impossibility for these to cover the whole ventilation variation range.

## Claims

1. A portable device for the survey of the breathing ventilation and of the oxygen consumption, comprising a system (6) for the survey of the gaseous volume, said system is mounted on a mask (M) with a tube wherethrough expired and inhaled air flows, a meter for the oxygen concentration and a control and radio transmission module for sending radio signals to a fixed reception and elaboration unit, said module being of small dimensions and weight so as to be portable for the person to be examined, characterized in that said gaseous volume survey system (6) consists of a turbine (7) placed in said tube and rotating at a speed proportional to the air volume striking the same, means for generating impulses with a rate proportional to the revolution number of the turbine and connected to a Schmitt-trigger amplifier (14), a pressure transducer (9) for recognizing the air flow annulment in the tube, whereupon the transmission of the impulses, deriving from the moment of inertia of said turbine (7) to the Schmitt trigger amplifier is prevented; the outlet of the Schmitt trigger is connected to a power amplifier (17) which controls the capacity of a sampling pump (4) for sampling air from said tube; said outlet is further connected to an integrator (16), controlling the amplification factor of said power amplifier (17) so that the sampling flow of said pump is proportional to the expiratory flow, and to a modulator (23) and to a radio transmitter (25); air sampled by the pump (4) is sent to a micro mixing chamber (3) with a polarographic electrode (2) for the measurement of the oxygen percentage and the output of the electrode is connected to said modulator (23) and to said transmitter (25).

2. A portable device for the survey of the ventilation according to claim 1, characterized in a temperature sensor (8) for the survey of the expired gases, the output thereof being amplified by an amplifier (18) and sent to the radio frequency modulator (23) and to the transmitter (25).

3. A portable device for the survey of the ventilation according to claim 1, characterized in that the output signal of transducer (9) is filtered by an electronic filter (11) and sent to a comparator (12) that controls a transistor (FET 13), the ON-OFF working whereof activates or disactivates the transmission of an impulse train to the Schmitt trigger (14) amplifier.

4. A portable device for the survey of the ventilation according to claim 1, characterized in the presence of auxiliary inlets (24) for auxiliary signals coming from other instruments like ECG (electrocardiographic signal), and also characterized in the transmission of said signals.

5. A portable device for the survey of the ventilation according to claim 1, characterized in the presence of a tape recorder or a digital recorder that can replace transmitter (25), so as to allow the late analysis of the memorized data.

6. A portable device for the survey of the ventilation according to claim 1, characterized in the presence of a recorder for the data in addition to transmitter (25).

## Patentansprüche

1. Tragbare Vorrichtung für die Vermessung der Atmungsventilation und des Sauerstoffverbrauchs, miteinschliessend ein System (6) für die Vermessung des gasförmigen Volumen, das genannte System ist auf eine Gesichtsmaske (M) aufgestellt mit einem Rohr durch dem die eingeatmete und ausgeatmete Luft strömt, einen Messer für Sauerstoffkonzentration und einen Uberprüfungs- und Radioübertragungsmodul um einer festen Empfangs- und Bearbeitungseinheit Radiosignale zu übersenden, der genannte Modul klein und leicht um für die prüfende Person tragbar sein zu können, dadurch gekennzeichnet, dass das genannte Vermessungssystem (6) des gasförmigen Volumen in eine im genannten Rohr gesetzte Turbine (7) besteht und die sich zu einer dem beträchtlichem Luftvolumen verhältnismässigen Geschwindigkeit dreht, Mitteln um Impulsen zu schaffen, zu einer der Anzahl von Drehungen der Turbine verhältnismässigen Geschwindigkeit, und dem Verstärker (4) des Schmitt-Triggers verbunden, einen Druckwandler (9) um die Annullierung der Luftströmung im Rohr zu vermessen, nachdem die Impulseübertragung, welche vom Trägheitsmoment der genannten Turbine (7) herkommt, zum Verstärker des Schmitt-Triggers behindert ist; der Ausgang des Schmitt-Triggers ist einem Stärkeverstärker (17) verbunden, welcher die Kapazität der Prüferpumpe (4), um die Luft vom genannten Rohr als Muster zu entnehmen, überprüft; der genannte Ausgang ist weiter einem Integrator (16) verbunden, welcher den Verstärkungsfaktor des genannten Stärkeverstärkers (17) überprüft, so dass die Probeentnahmeströmung der genannten Pumpe der Expirationsströmung verhältnismässig ist, und einem Modulator (23) und einem Radioübertrager (25); die Luft von der Pumpe (4) bemustert wird einer Mischungsmikrokammer (3) übersendet, mit einer Polarograph-Elektrode für die Messung des Sauerstoffprozentsatz und der Ausgang der Elektrode ist dem genannten Modulator (23) und dem genannten Sender (25) verbunden.

2. Tragbare Vorrichtung für die Vermessung der Ventilation, nach Patentanspruch 1, gekennzeichnet durch den Temperatursensor (8) für die Vermessung der Temperatur der ausgeatmeten Gase, von welchen der Austritt vom Verstärker (18) verstärkt wird und dem Radiofrequenzmodulator (23) und dem Radiosender (25) übersendet wird.

3. Tragbare Vorrichtung für die Vermessung der Ventilation nach Patentanspruch 1, dadurch gekennzeichnet, dass das Ausgabesignal des Wandlers (9) vom elektronischem Sieb (11) gesieben und dem Vergleicher (12) übersendet ist, welcher den Transistor FET (13) führt, dessen Bstrieb ON - OFF dem Schmitt-Trigger Verstärker (14) die Ubertragung des Impulsezugs befähigt oder unfähig macht.

4. Tragbare Vorrichtung für die Vermessung der Ventilation, nach Patentanspruch 1, gekennzeichnet durch die Anwesenheit der Hilfseingänge (24) für von anderen Instrumenten herkommende Hilfssignale, welche ECG (Elektrokardiographiesignal) und auch von der Ubertragung der genannten Signale gekennzeichnet.

5. Tragbare Vorrichtung für die Vermessung der Ventilation, nach Patentanspruch 1, gekennzeichnet durch die Anwesenheit eines Magnetband- oder Digitalaufnahmegerätes, geeignet, den Sender (25) zu ersetzen, um somit die verzögerte Analyse der gespeicherten Daten zu gestatten.

6. Tragbare Vorrichtung für die Vermessung der Ventilation, nach Patentanspruch 1, gekennzeichnet durch die Anwesenheit eines Datenregistrierapparates, in Zusatz zu dem Sender (25).

## Revendications

1. Dispositif portatif pour le relevé de la ventilation respiratoire et de la consommation de oxygène, comprenant un système (6) pour le relevé du volume gazeux, ledit système est monté sur un masque (M) facial avec un tuyau au moyen duquel coule l'air inspiré et expiré, un mesureur pour concentration de oxygène et un module de contrôle et transmission radio pour envoyer des signals radio à une unité fixe de réception et de élaboration, ledit module en étant de petits dimension et poids de manière à être portatif pour le sujet à examiner, caractérisé en ce que ledit système (6) de relevé du volume gazeux consiste en une turbine (7) placée dans ledit tuyau et qui tourne à une vitesse proportionnelle au volume d'air considérable, moyens pour produire des impulsions avec une vitesse proportionnelle au nombre de tours de la turbine et joint à l'amplificateur (4) du trigger de Schmitt, un transducteur de pression (9) pour relever l'annulation de l'écoulement d'air dans le tuyau, après que la transmission des impulsions, dérivant du moment d'inertie de ladite turbine (7) à l'amplificateur du trigger de Schmitt est entravée; la sortie du trigger de Schmitt est jointe à un amplificateur de puissance (17) qui contrôle la capacité de la pompe échantillonneur (4) pour échantillonner air par ledit tuyau; ladite sortie est ultérieurement jointe à un intégrateur (16), qui contrôle le facteur de amplification dudit amplificateur de puissance (17), de sorte que l'écoulement d'échantillonnage de ladite pompe est proportionnel à l'écoulement d'expiration, et à un modulateur (23) et à un transmetteur radio (25); l'air échantillonné par la pompe (4) vient envoyé à une micro-chambre de mélange (3) avec une électrode (2) polarographe pour le mesurage du pourcentage d'oxygène et la sortie de l'électrode est jointe audit modulateur (23) et audit transmetteur (25).

2. Dispositif portatif pour le relevé de la ventilation, selon la revendication 1, caractérisé par le mesureur (8) de température pour le relevé de la température des gaz expirés dont la sortie vient amplifiée par l'amplificateur (18) et envoyée au modulateur à radiofréquence (23) et à l'émetteur (25).

3. Dispositif portatif pour le relevé de la ventilation, selon la revendication 1, caractérisé en ce que le signal de sortie du transducteur (9) a filtré par le filtre électronique (11) et envoyé au comparateur (12) qui pilote le transistor FET (13) dont le fonctionnement ON - OFF habilito ou déshabilite la transmission du train d'impulsions à l'amplificateur à trigger de Schmitt (14).

4. Dispositif portatif pour le relevé de la ventilation, selon la revendication 1, caractérisé par la présence des les entrées auxiliaires (24) pour signals auxiliaires provenants d'autres instruments tels que ECG (signal d'électrocardiographie) et caractérisé aussi par la transmission desdits signals.

5. Dispositif portatif pour le relevé de la ventilation, selon la revendication 1, caractérisé par la présence d'un enregistreur à bande magnétique ou digitale, apte à remplacer le transmetteur (25), de manière à permettre l'analyse en renvoi des données mémorisées.

6. Dispositif portatif pour le relevé de la ventilation, selon la revendication 1, caractérisé par la présence d'un enregistreur des données en addition au transmetteur (25).

## FIG.1

M

UF

FIG.2

EP 0 196 396 B1

FIG.3